# EUROPEAN PATENT APPLICATION

(11) **EP 2 679 203 A1**
(43) Date of publication of application: **01.01.2014**
(21) Application number: 13173134.1
(22) Date of filing: 21.06.2013
(51) Int. Cl.: A61F 5/01

(54) **Anti-migration pad for an orthopedic brace**

(30) Priority: 27.06.2012 US 201213534843
(71) Applicant: Breg, Inc., Carlsbad, CA 92010 (US)
(72) Inventor: Cardinali, Mathew, Berwick, Maine 03901 (US)
(74) Representative: Meldrum, David James

(57) **Abstract**

A pad for an associated orthopedic brace has a laminate structure including a first layer and a second layer. The first layer is formed from a pliant material having a plurality of interstitial perforations formed therein. The second layer abuts the first layer and is formed from an elastic pliant material which projects outward through the perforations of the first layer to form nubs that are raised relative to the first layer. The nubs have a relatively high friction coefficient when coupled with the skin of the associated orthopedic brace wearer. As such, the nubs, in combination with the first layer, define an uneven skin contact surface which renders the pad and associated brace resistant to migration when mounted on the body of a wearer.

## Description

### BACKGROUND

The present disclosure relates generally to orthopedic braces, and more particularly to a pad which is positioned between an associated orthopedic brace and the body of a wearer.

Orthopedic braces embody a broad range of structures, each having the common purpose of supporting and/or stabilizing a region of the body such as a skeletal joint when the brace is appropriately positioned on the body of a user. The orthopedic brace generally serves either a preventative role or a remedial role. For example, in a preventative role the brace may provide added support and stability to a healthy skeletal joint, thereby reducing the risk of injury when the joint is subjected to undue stress. In a remedial role, the brace may support and stabilize a skeletal joint which has been weakened by injury or other infirmity, thereby reinforcing the joint and reducing the risk of further injury while the joint is rehabilitated.

Conventional orthopedic braces typically have a support member which is positioned in abutment with a region of the body which is intended to benefit from the added support or stability provided by the brace. In cases where the orthopedic brace is intended to support and/or stabilize a skeletal joint, the brace typically has multiple support members which are positioned on either side of the skeletal joint. The support members may be dynamically interconnected by a hinge which is positioned at the skeletal joint to mimic the motion of the joint. For example, a conventional knee brace commonly includes a rigid frame having an upper support member positioned adjacent to the upper leg of the wearer and a lower support member positioned adjacent to the lower leg of the wearer. A rotational hinge is positioned adjacent to the knee joint of the wearer which dynamically interconnects the upper and lower support members enabling controlled pivotal movement of the knee joint when the wearer engages in activity or rehabilitative therapy.

Orthopedic braces are typically secured to the body of the wearer at a desired mounting position by securing means such as straps which engage both the body and the support members of the brace. Orthopedic braces are also typically provided with padding which cushions the body of the wearer from the support members of the brace when the brace is mounted on and secured to the body of the wearer.

### SUMMARY

The present disclosure recognizes the importance and need for an orthopedic brace which retains its desired mounting position on the body of a wearer during use. Migration of the brace away from the desired mounting position during use undesirably diminishes both the functional performance of the brace and the comfort of the wearer. Accordingly, according to one aspect, there can be provided an orthopedic brace which retains it desired mounting position on the body of a wearer during use. According to another aspect, there can be provided an anti-migration pad which is positioned between a support member of an orthopedic brace and the body of the wearer which resists migration of the brace from the desired mounting position during use when the brace is mounted on the body of the wearer. Particular and preferred aspects are set out in the appended independent and dependent claims.

One example in accordance with the present teachings provides a pad for an associated orthopedic brace. The pad comprises a laminate having a skin contact surface. The laminate has a first layer and a second layer. The first layer is formed from a first material which is preferably pliant such as a perforated cloth or more particularly a mesh. In accordance with one embodiment, the first material is substantially non-stretchable. In any case, the first material has a plurality of interstitial perforations formed therein and has planar faces defined by continuous planar expanses of the pliant material extending between the interstitial perforations.

The second layer is formed from a second material which is preferably pliant and elastic such as an elastomer, gel or foam and more particularly a low-density closed-cell polymer foam. In accordance with one embodiment, the second material is substantially stretchable. In any case, the second layer of the laminate abuts the first layer. The second material projects outward from the second layer through the interstitial perforations of the first layer forming raised nubs. The skin contact surface of the laminate comprises in combination the nubs of the second layer and the planar faces of the first layer. The surface is uneven with the nubs being raised relative to the planar faces.

The second material from which the nubs are formed and the skin of an associated orthopedic brace wearer preferably have a higher friction coefficient relative to the first material from which the planar expanses are formed and the skin of the wearer. The second material also preferably has a lower heat distortion temperature relative to the first material.

Another example in accordance with the present teachings provides a method of fabricating an orthopedic brace pad. The method comprises energizing and pressurizing steps followed by de-energizing and de-pressurizing steps. As such, the method includes energizing a laminate having a first sheet of a perforated first material and a second sheet of a second material which abuts the first sheet. The second sheet is energized to a sufficient degree to substantially soften the second material to a substantially deformable state. In accordance with one embodiment, the laminate is energized by heating the laminate and more particularly by heating the laminate to a temperature above the heat distortion temperature of the second material which is preferably lower than the heat distortion temperature of the first material. In any case, sufficient pressure is applied to the softened second sheet to extrude the second material through perforations in the perforated first material of the first sheet. The laminate is then de-energized and de-pressurized resulting in an orthopedic brace pad having a plurality of nubs formed from the second material. The nubs and first sheet comprise in combination an uneven skin contact surface of the pad with the nubs being raised relative to the first sheet.

The teachings provided by the present disclosure will be further understood from the accompanying drawings and description.

### BRIEF DESCRIPTION OF THE DRAWINGS

FIG. 1 is a perspective view of a knee brace and associated anti-migration pads.
FIG. 2 is partial cross-sectional view of the upper pad and overlaying support element of FIG. 1, which is taken along line 2-2.

The above-listed drawing figures illustrate one or more embodiments of the present teachings by way of example and not by way of limitation. Common reference characters may be used among the different drawing figures to indicate the same or similar structural elements. However, the terms "an embodiment", "one embodiment", "an alternate embodiment", or similar such terminology appearing below do not necessarily indicate the same embodiment of the teachings when there are multiple occurrences of the same term.

### DETAILED DESCRIPTION

The present disclosure provides teachings in relation to a pad for an associated orthopedic brace. The orthopedic brace having utility herein is configured for mounting on the body of a person at a specific position where supplemental support is desired. The orthopedic brace includes one or more support elements which abut the body and provide supplemental support to the body at the desired mounting position. A typical mounting position of the orthopedic brace is across a joint of the wearer for which supplemental support is desired, such as a knee, ankle, hip, elbow, wrist or shoulder.

In accordance with one embodiment, the orthopedic brace, with which the pad of the present teachings is associated, may be considered as being as a substantially rigid brace, wherein its one or more support elements are substantially hard rigid structures fabricated from such substantially hard rigid materials as metals, plastics, fiberglass, composites or the like. An example of a rigid orthopedic brace is a knee brace having a hinged frame which is mounted on the leg surrounding the knee joint and secured to the leg by straps. The frame is constructed from one or more rotational hinges and a plurality of substantially rigid members functioning as rigid support elements. The substantially rigid members are dynamically joined to one another at the knee joint by the rotational hinges.

In accordance with another embodiment, the orthopedic brace, with which the pad of the present teachings is associated, may be considered as being a substantially soft brace, wherein its one or more support elements are substantially soft pliant, i.e., flexible, structures fabricated from such substantially soft pliant materials as natural or synthetic cloths or fabrics, foams, laminates or the like. An example of a soft orthopedic brace is a knee brace constructed in a continuous tubular configuration from a pliant elastic material functioning as a soft support element. The knee brace slides over and encloses the knee joint in the manner of a sleeve.

In accordance with yet another embodiment, the orthopedic brace, with which the pad of the present teachings is associated, may be considered as being a combination brace having both rigid and soft support elements. An example of a combination orthopedic brace is a knee brace having a sheet of soft pliant material which wraps around the leg at the knee joint and releasably fastens to itself, thereby enclosing the knee joint and functioning as a soft support element. The sheet is provided with one or more longitudinal pockets. A hinged rigid support member is inserted into, and retained by, each longitudinal pocket, thereby functioning as a rigid support element.

In any case, whether rigid or soft, each support element of the orthopedic brace, with which the pad of the present teachings is associated, is two-sided. One side of the support element is termed an inside surface and its other opposite side is termed an outside surface. The inside surface of the support element is intended to face toward the body of the wearer at the desired mounting position and to face away from the surrounding external environment when the orthopedic brace is mounted on the body. Conversely, the outside surface of the support element is intended to face toward the surrounding external environment and to face away from the body of the wearer at the desired mounting position when the orthopedic brace is mounted on the body.

The pad of the present teachings is configured to be positioned between a support element of the associated orthopedic brace and the body of the wearer at the desired mounting position when the orthopedic brace is mounted on the body. The pad, like the support elements, is two-sided with one side of the pad being termed an inside surface and the opposite side being termed an outside surface. The inside surface of the pad is intended to face away from the inside surface of the associated support element and to face toward the body of the wearer and engage the skin at the desired mounting position when the orthopedic brace is mounted on the body. Conversely, the outside surface of the pad is intended to face away from the body of the wearer at the desired mounting position and to face toward and engage the inside surface of the associated support element when the orthopedic brace is mounted on the body.

The pad of the present teachings can provide *inter alia* a shielding/cushioning function and/or a high-friction/anti-migration function when the orthopedic brace is mounted on the body with the pad positioned between the associated support element of the orthopedic brace and the body of the wearer as described above. Specifically, the pad performs the shielding/cushioning function by shielding the body of the wearer from direct contact with the support element of the orthopedic brace when the orthopedic brace is mounted on the body. Accordingly, the pad cushions body of the wearer from the support element of the brace. This function may be particularly appropriate when the support element is a substantially rigid support member, but can also have utility with a soft or combination brace.

The pad performs the high-friction/anti-migration function by constructing the inside surface of the pad from materials which are configured to provide the pad and skin of the body of the wearer in combination with a relatively high coefficient of friction across their points of contact. Accordingly, the pad resists migration of the orthopedic brace from its desired mounting position on the body during use to maintain good performance of the brace.

A specific example of a pad of the present teachings and an associated orthopedic brace are described below with reference to the drawings for purposes of illustrating an example embodiment of the present teachings. However, it understood from the above that the present disclosure is not limited to this example embodiment, but is generally applicable to anti-migration pads that fall within the spirit and scope of the present teachings which can be fitted to a broad range of available rigid, soft or combination orthopedic braces for any joint or body part.

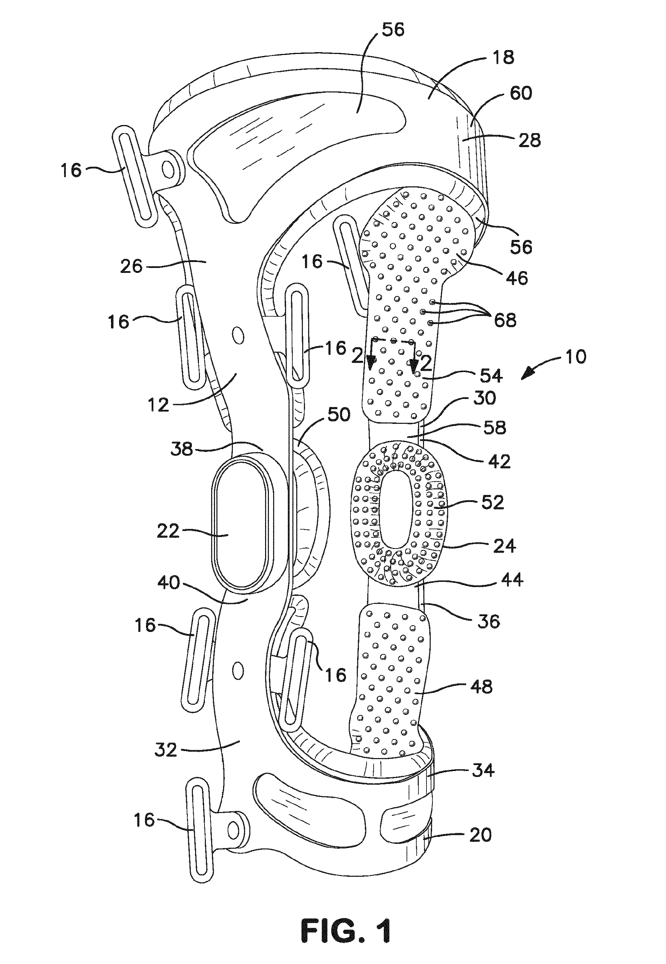

Referring initially to FIG. 1, a rigid knee brace of the type generally described above is shown and designated 10. For purposes of illustration, the rigid knee brace 10 is configured for securing to a right leg of a wearer. However, it is readily apparent to the skilled artisan from the teaching provided herein that the rigid knee brace 10 can be adapted for securing to the opposite leg of the wearer. There are a number of relative terms defined below which are used in the following description to distinguish various elements of the rigid knee brace 10 from one another, but which are not to be construed as limiting the scope of the present teachings. The relative terms "medial" and "lateral" describe the relative proximity of certain elements to the central longitudinal axis of the body of the wearer when the rigid knee brace 10 is secured to a leg of the wearer. A "medial" element is closer to the central longitudinal axis of the body, while a "lateral" element is further from the central longitudinal axis of the body. The terms "upper" and "lower" describe the position of certain elements as being either above or below the rotational hinges of the rigid knee brace 10 and correspondingly either above or below the knee joint. An "upper" element is above the rotational hinges and knee joint, while a "lower" element is below the rotational hinges and knee joint.

Most conventional rigid knee braces include at least the following common elements: a hinged frame, a plurality of straps and strap retainers, and brace padding. The present rigid knee brace 10 likewise includes all of these common elements although only the hinged frame 12, strap retainers 16, and brace padding are shown in FIG. 1. The straps associated with the strap retainers 16 are omitted for clarity, but the placement and function of the omitted straps on the rigid knee brace 10 is, nevertheless, readily apparent to the skilled artisan. It is further noted that brace padding is generally known for use in conventional rigid knee braces to perform a cushioning function. However, the specific brace padding shown in FIG. 1 comprises a plurality of novel anti-migration pads 46, 48, 50, 52 embodying the present teachings which are not known in the state of the art. The specific novel features of the anti-migration pads 46, 48, 50, 52 will be described in greater detail below.

The hinged frame 12 of the rigid knee brace 10 includes an upper frame assembly 18, a lower frame assembly 20, a lateral rotational hinge assembly 22, and a medial rotational hinge assembly 24. The upper frame assembly 18 is an integral structure which combines multiple support elements, namely, a lateral upper longitudinal support 26, an upper cuff support 28, and a medial upper longitudinal support 30. The lower frame assembly 20 is similarly an integral structure which combines multiple support elements, namely, a lateral lower longitudinal support 32, a lower cuff support 34, and a medial lower longitudinal support 36.

The upper and lower frame assemblies 18, 20 are laterally connected to one another by the lateral rotational hinge assembly 22 which is positioned at a central horizontal rotational axis of the frame 12. In particular, the lateral rotational hinge assembly 22 in cooperation with the lateral upper and lower longitudinal supports 26, 32 effects rotational connection of the upper and lower frame assemblies 18, 20. The lateral upper longitudinal support 26 is an elongate arm having a lateral upper hinge end 38 and the lateral lower longitudinal support 32 is similarly constructed having a lateral lower hinge end 40. The lateral upper and lower hinge ends 38, 40 are specifically configured to cooperatively engage one another and to engage the lateral rotational hinge assembly 22. Thus, the lateral rotational hinge assembly 22 enables rotational displacement of the lateral upper and lower longitudinal supports 26, 32 about the central rotational axis of the frame 12 between positions of extension and flexion.

The upper and lower frame assemblies 18, 20 are similarly medially connected to one another by the medial rotational hinge assembly 24 positioned at the central horizontal rotational axis of the frame 12. In particular, the medial rotational hinge assembly 24 effects rotational connection of the upper and lower frame assemblies 18, 20 in cooperation with the medial upper and lower longitudinal supports 30, 36. The medial upper longitudinal support 30 is an elongate arm having a medial upper hinge end 42 and the medial lower longitudinal support 36 is similarly constructed having a medial lower hinge end 44. The medial upper and lower hinge ends 42, 44 are specifically configured to cooperatively engage one another and to engage the medial rotational hinge assembly 24. Thus, the medial rotational hinge assembly 24 enables rotational displacement of the medial upper and lower longitudinal supports 30, 36 about the central rotational axis of the frame 12 between positions of extension and flexion.

The lateral and medial rotational hinge assemblies 22, 24 are each conventional rotational hinge assemblies. One such conventional rotational hinge assembly having utility herein is disclosed in U.S. Patent No. 5,772,618, which is incorporated herein by reference. Additional example conventional hinge assemblies having utility herein are disclosed in U.S. Patent Nos. 401,933; 4,481,941; 5,672,152; and 5,827,208. Alternatively, one or both of the lateral and medial rotational hinge assemblies 22, 24 can be constructed in a manner which enables an osteoarthritis treatment function as is well known to one of ordinary skill in the art.

The anti-migration pads 46, 48, 50, 52 are more specifically termed an upper pad 46, a lower pad 48, a lateral condyle pad 50 and a medial condyle pad 52, respectively. The upper pad 46 is configured in correspondence with the configuration of the upper frame assembly 18. Although the upper pad 46 is a unitary pad, it can be considered as comprising a lateral upper segment, an upper cuff segment and a medial upper segment, which abut and substantially cover the inside surfaces of the corresponding rigid support elements of the upper support frame 18, namely, the lateral upper longitudinal support 26, upper cuff support 28, and medial upper longitudinal support 30, respectively. In particular, the lateral upper segment has an outline corresponding to the outline of the lateral upper longitudinal support 26 and can be flexed from its planar construction into cooperative engagement with the curved contours of the inside surface of the lateral upper longitudinal support 26. The upper cuff segment has an outline corresponding to the outline of the upper cuff support 28 and can be flexed from its planar construction into cooperative engagement with the curved contours of the inside surface of the upper cuff support 28. The medial upper segment has an outline corresponding to the medial upper longitudinal support 30 and can be flexed from its planar construction into cooperative engagement with the curved contours of the inside surface of the medial upper longitudinal support 30.

The lower pad 48 is similarly configured in correspondence with the configuration of the lower frame assembly 20. Although the lower pad 48 is likewise a unitary pad, it can be considered as comprising a lateral lower segment, a lower cuff segment and a medial lower segment, which abut and substantially cover the inside surfaces of the corresponding rigid support elements of the lower frame assembly 20, namely, the lateral lower longitudinal support 32, lower cuff support 34, and medial lower longitudinal support 36, respectively. In particular, the lateral lower segment has an outline corresponding to the outline of the lateral lower longitudinal support 32 and can be flexed from its planar construction into cooperative engagement with the curved contours of the inside surface of the lateral lower longitudinal support 32. The lower cuff segment has an outline corresponding to the outline of the lower cuff support 34 and can be flexed from its planar construction into cooperative engagement with the curved contours of the inside surface of the lower cuff support 34. The medial lower segment has an outline corresponding to the medial lower longitudinal support 36 and can be flexed from its planar construction into cooperative engagement with the curved contours of the inside surface of the medial lower longitudinal support 36.

The lateral condyle pad 50 is configured in correspondence with the lateral rotational hinge assembly 22. Accordingly, the lateral condyle pad 50 abuts and substantially covers the inside surface of the corresponding lateral rotational hinge assembly 22. The medial condyle pad 52 is similarly configured in correspondence with the medial rotational hinge assembly 24. Accordingly, the medial condyle pad 52 abuts and substantially covers the inside surface of the corresponding medial rotational hinge assembly 24. Since the lateral and medial rotational hinge assemblies 22, 24 have substantially the same configuration, the lateral and medial condyle pads 50, 52 likewise have substantially the same configuration.

Although it is apparent from the above that some differences exist between the configurations of the respective anti-migration pads 46, 48, 50, 52, all of the anti-migration pads preferably share a common construction. Accordingly, an illustrative construction of the upper pad 46 is described below by way of example with the understanding that this same description may likewise be applied to the construction of the lower pad 48 as well as the lateral and medial condyle pads 50, 52.

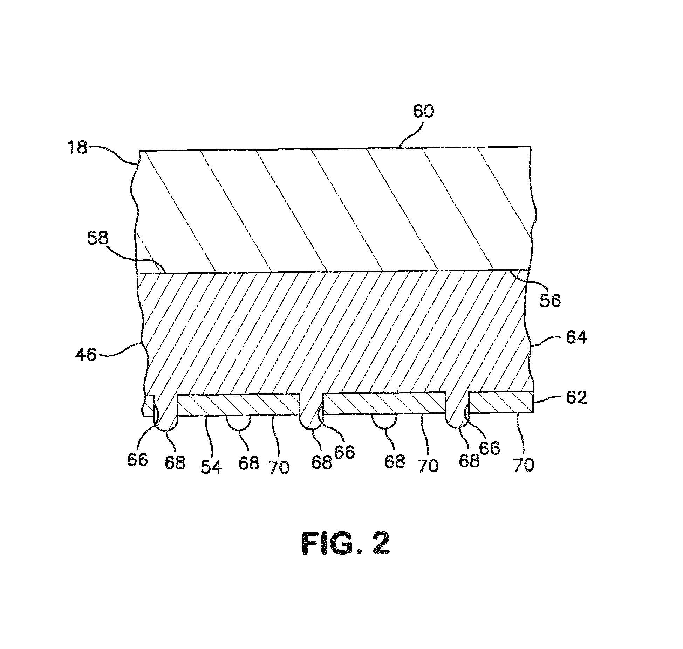

With continuing reference to FIG. 1 and additional reference to FIG. 2, the upper pad 46 has an inside surface 54 on one side and an outside surface 56 on its opposite side. The overlaying upper frame assembly 18 likewise has an inside surface 58 on one side and an outside surface 60 on its opposite side. When the knee brace 10 is mounted on the leg about the knee joint, the inside surface 54 of the upper pad 46 faces toward the thigh of the wearer (not shown) and engages the skin on the thigh. Conversely, the outside surface 56 of the upper pad 46 faces toward and engages the inside surface 58 of the upper frame assembly 18 when the knee brace 10 is mounted on the leg. The outside surface 60 of the upper frame assembly 18 faces toward the surrounding external environment when the knee brace 10 is mounted on the leg.

The upper pad 46 has a laminate construction comprising a first layer 62 and a second layer 64. In accordance with one embodiment, the first layer 62 is generally considered as an inside layer and the second layer 64 is generally considered as an outside layer. The first layer 62 is a relatively thin planar sheet of a pliant material having a plurality of perforations 66 formed therein at spaced-apart intervals. The planar sheet of the first layer 62 may suitably be a mesh, wherein the perforations 66 are distributed across at least a portion, if not all, of the sheet in a substantially uniform pattern. An example of a suitable pliant material of the first layer is a perforated synthetic cloth such as a nylon mesh or the like. Although the material of the first layer 62 may suitably be pliant, it may also suitably be substantially non-stretchable.

The second layer 64 is a substantially thicker sheet of a pliant elastic material, e.g., having a thickness on the order of about 1/4 inches or so. An example of a suitably pliant elastic material of the second layer 64 is a compressible elastomer, gel or foam. An example foam is a low-density closed-cell polymer foam. An example pliant elastic material of the second layer 64 is also relatively tacky to the touch which can thereby enhance the degree of friction between the material of the second layer 64 and the skin of the wearer of the knee brace 10 when the inside surface 54 of the upper pad 46 and the skin contact one another. In any case, the material of the second layer 64 may suitably have a substantially lower heat distortion temperature (HDT), i.e., heat deflection temperature, than the material of the first layer 62.

The inside surface 54 of the upper pad 46 is considered as uneven due to the presence of a plurality of raised nubs 68 positioned on the inside surface 54 in correspondence with the position of the perforations 66 of the first layer 62. The nubs 68 are low-profile structures which are continuous with, and formed from, the same material as the sheet of the second layer 64. The nubs 68 project outward from the sheet of the second layer 64, through the perforations 66 of the first layer 62 and past the planar faces 70 of the first layer 62. The planar faces 70 are defined as the limited planar expanses of continuous pliant material, e.g., cloth, extending between the interstitial perforations 66. As such, the uneven inside surface 54 of the upper pad 46 comprises in combination the raised nubs 68 of the second layer 64 and the depressed planar faces 70 of the first layer 62.

The raised position of the nubs 68 causes the nubs 68 to be the initial and primary points of contact between the skin of a wearer and the knee brace 10 when the knee brace 10 is mounted on the leg. Since the material of the nubs 68 in combination with the skin has a high coefficient of friction relative to the material of the first layer 62 in combination with the skin, there is a relatively high degree of friction between the skin and the upper pad 46. The tackiness of the material forming the nubs 68 also contributes to the degree of friction between the nubs 68 and skin. This high degree of friction causes the nubs 68 to effectively "grip" the skin when the nubs 68 and skin come into contact with one another.

The upper pad 46 is preferably static relative knee brace 10 as the result of releasable or fixable attachment of the upper pad 46 to the upper frame assembly 18 in a manner described below. Consequently, the gripping effect of the nubs 68 can significantly impede movement of the knee brace 10 relative to the leg once the knee brace 10 is mounted on the leg at the desired mounting position about the knee joint. As a result undesirable slippage and displacement of the knee brace 10 from the desired mounting position about the knee joint is diminished, if not avoided altogether, and improved functional performance of the knee brace 10 for its intended purpose of supporting and/or stabilizing the knee joint is achieved.

In contrast to the inside surface 54 of the upper pad 46, the outside surface 56 of the upper pad 46 may suitably be smooth and comprise in its entirety the planar face of the second layer 64 which is on the opposite side of the second layer 64 from the nubbed face. The outside surface 56 of the upper pad 46 may suitably be releasably or fixably attachable to the inside surface 58 of the upper frame assembly 18 which is likewise relatively smooth. Releasable attachment of the upper pad 46 and upper frame assembly 18 to one another is effected by releasable fastening means, such as hook and loop fasteners commercially available under the trade name VELCRO. In accordance with one embodiment of the releasable fastening means, a sheet of loop material (not shown) is bonded or otherwise affixed to the smooth planar face of the second layer 64 by substantially any permanent fastening means such as thermal or ultrasonic welding, gluing, sewing or the like to form the outside surface 56 of the upper pad 46. One or more patches of hook material (not shown) are also bonded or otherwise affixed to the inside surface 58 of the upper frame assembly 18. As an alternative to releasable attachment, fixable attachment of the upper pad 46 and upper frame assembly 18 to one another can be effected by gluing or some other similar permanent fastening means. It is readily apparent that releasable or fixable attachment of the lower pad 48 to the lower frame assembly 20 and the lateral and medial condyle pads 50, 52 to the lateral and medial rotational hinge assemblies 22, 24, respectively, can be effected in substantially the same manner as described above with respect to the upper pad 46 and upper frame assembly 18.

The above description applies to specific case where anti-migration pads of the present teachings are configured in correspondence with a particular embodiment of rigid support elements. However, it is readily apparent to one of ordinary skill in the art that the instant teaching for configuring anti-migration pads in correspondence with the shape and size of the rigid support elements specifically disclosed herein can be applied generally to configure anti-migration pads in correspondence with substantially any alternately shaped and/or sized support element, either rigid or soft, and falls within the scope of the present disclosure.

The above description also applies to a specific case where anti-migration pads of the present teachings are releasably or fixably attached to rigid support elements. The present teachings additionally encompass releasable or fixable attachment of anti-migration pads, as described herein, to soft support elements of a soft orthopedic brace or a combination orthopedic brace. The releasable fastening means, such as hook and loop fasteners, and the permanent fastening means, such as glue or other bonders, described above with respect to rigid support elements can be similarly employed to releasably or fixably attach anti-migration pads to soft support elements. In addition sewing can be used as an alternate permanent fastening means for fixably attaching anti-migration pads to soft support elements.

The integrated laminate construction of the anti-migration pads of the present teachings can be achieved by a number of different fabrication methods. However, an example embodiment of an illustrative anti-migration pad fabrication method is described below. The practitioner initiates the fabrication method by selecting a first sheet of perforated pliant material which will form the first layer 62 of the anti-migration pad and a second sheet of pliant elastic material which will form the second layer 64 of the anti-migration pad. The first and second sheets both preferably have substantially uniform thicknesses. However, the uniform thickness of the second sheet may suitably be substantially greater than the uniform thickness of the first sheet. It is further noted that at the outset the faces of both sides of the second sheet may suitably be relatively smooth.

A planar unitary laminate of substantially uniform thickness is formed by stacking the first and second sheets atop one another such that the abutting surfaces of the first and second sheets continuously engage one another. The first and second sheets are substantially permanently and continuously bonded together across substantially the entirety of their engaged surfaces by substantially any conventional bonding means such as gluing, thermal welding, ultrasonic welding, or the like. A blank is cut out of the resulting laminate based on a pattern matching a desired outline of the pad, which typically corresponds to the outline of an associated support element as described above.

The nubs 60 are formed on the face of the blank on which the first sheet is bonded by thermally or ultrasonically energizing and compressing the blank. The resultant heat or ultrasonic energy softens the material of the second sheet. A compression tool is used to apply compression to the side of the energized blank opposite the side on which the first sheet is bonded. The resultant pressure in the direction of the first sheet causes the softened material to plastically deform and be extruded outward through the perforations 66 in the first sheet. Once the desired nub size and configuration are achieved, compression is terminated and the resulting anti-migration pad is cooled such that the desired nubs 60 are irreversibly permanently formed on the inside surface 54 of the anti-migration pad. The perimeter of the anti-migration pad may also be trimmed further to more precisely match the desired pad outline, thereby completing fabrication of the anti-migration pad. It is apparent to one of ordinary skill in the art that the practitioner exercises process control over the present fabrication method by selecting and controlling certain operating parameters, including, the materials of the first and second sheets and the design features of the compression tool as well as the time, temperature and pressure of the energizing/compression steps.

It is alternately within the scope of the present teachings to modify the above-described fabrication method by switching the sequence of the stacking step, the bonding step and/or the cutting step, so that the sheets are cut to the outline of the pad before the sheets are stacked and/or bonded. In any case, it is noted that the resulting anti-migration pad typically maintains a planar configuration until manually flexed into a contoured configuration for engagement with the respective support element. If the anti-migration pad is subsequently disengaged from the support element, it readily returns to its planar configuration.

Therefore, from one viewpoint, there has been described a pad for an associated orthopedic brace that has a laminate structure including a first layer and a second layer. The first layer is formed from a pliant material having a plurality of interstitial perforations formed therein. The second layer abuts the first layer and is formed from an elastic pliant material which projects outward through the perforations of the first layer to form nubs that are raised relative to the first layer. The nubs have a relatively high friction coefficient when coupled with the skin of the associated orthopedic brace wearer. As such, the nubs, in combination with the first layer, define an uneven skin contact surface which renders the pad and associated brace resistant to migration when mounted on the body of a wearer.

Further example feature combinations provided by the present disclosure are set out in the following numbered clauses:
Clause 1. A pad for an associated orthopedic brace comprising: a laminate having a skin contact surface; a first layer of said laminate formed from a first material having a plurality of interstitial perforations formed therein and planar faces defined by continuous planar expanses of said pliant material extending between said interstitial perforations; and a second layer of said laminate abutting said first layer formed from a second material, wherein said second material projects outward from said second layer through said interstitial perforations of said first layer forming raised nubs, further wherein said skin contact surface of said laminate comprises in combination said nubs and said planar faces and is uneven, said nubs being raised relative to said planar faces.
Clause 2. The pad of clause 1, wherein said first material is pliant.
Clause 3. The pad of clause 1 or 2, wherein said first material is a perforated cloth.
Clause 4.The pad of clause 1, 2 or 3, wherein said first material is a mesh.
Clause 5.The pad of any preceding clause, wherein said first material is substantially non-stretchable.
Clause 6. The pad of any preceding clause, wherein said second material is pliant and elastic.
Clause 7. The pad of any preceding clause, wherein said second material is an elastomer, gel or foam.
Clause 8. The pad of any preceding clause, wherein said second material is a low-density closed-cell polymer foam.
Clause 9. The pad of any preceding clause, wherein said second material is substantially stretchable.
Clause 10. The pad of any preceding clause, wherein said second material and the skin of an orthopedic brace wearer have a higher friction coefficient relative to the first material and the skin of an orthopedic brace wearer.
Clause 11. The pad of any preceding clause, wherein said second material has a lower heat distortion temperature relative to said first material.
Clause 12. A pad for an associated orthopedic brace comprising: a laminate having a skin contact surface; a first layer of said laminate formed from a pliant cloth having a plurality of interstitial perforations formed therein and planar faces defined by continuous planar expanses of said pliant material extending between said interstitial perforations; and a second layer of said laminate abutting said first layer formed from a pliant elastic foam, wherein said foam projects outward from said second layer through said interstitial perforations of said first layer forming raised nubs, further wherein said skin contact surface of said laminate comprises in combination said nubs and said planar faces and is uneven, said nubs being raised relative to said planar faces.
Clause 13. A method of fabricating an orthopedic brace pad comprising: energizing a laminate having a first sheet of a perforated first material and a second sheet of a second material abutting said first sheet to a sufficient degree to substantially soften said second material to a substantially deformable state; applying sufficient pressure to said softened second sheet to extrude said second material through perforations in said perforated first material of said first sheet; and de-energizing and de-pressurizing said laminate resulting in an orthopedic brace pad having a plurality of nubs formed from said second material, wherein said nubs and said first sheet comprise in combination an uneven skin contact surface of said pad, said nubs being raised relative to said first sheet.
Clause 14. The method of clause 13, wherein said laminate is energized by heating said laminate.
Clause 15. The method of clause 13 or 14, wherein said laminate is energized by heating said laminate to a temperature above the heat distortion temperature of said second material.
Clause 16. The method of clause 13, 14 or 15, wherein said first material is a perforated cloth.
Clause 17. The method of any of clauses 13 to 16, wherein said second material is an elastomer, gel or foam.
Clause 18. The method of any of clauses 13 to 17, wherein said second material is a low-density closed-cell polymer foam.
Clause 19. The method of any of clauses 13 to 18, wherein said second material has a lower heat distortion temperature relative to said first material.
Clause 20. The method of any of clauses 13 to 19, wherein said second material and the skin of an orthopedic brace wearer have a higher friction coefficient relative to the first material and the skin of an orthopedic brace wearer.

While the forgoing preferred embodiments of the present disclosure have been described and shown, it is understood that alternatives and modifications, such as those suggested and others, may be made thereto and fall within the spirit and scope of the [resent teachings.

## Claims

1. A pad for an associated orthopedic brace comprising:
a laminate having a skin contact surface;
a first layer of said laminate formed from a first material having a plurality of interstitial perforations formed therein and planar faces defined by continuous planar expanses of said pliant material extending between said interstitial perforations; and
a second layer of said laminate abutting said first layer formed from a second material, wherein said second material projects outward from said second layer through said interstitial perforations of said first layer forming raised nubs, further wherein said skin contact surface of said laminate comprises in combination said nubs and said planar faces and is uneven, said nubs being raised relative to said planar faces.

2. The pad of claim 1, wherein said first material is pliant.

3. The pad of claim 1 or 2, wherein said first material is one or more selected from the group comprising: a perforated cloth; and a mesh.

4. The pad of any preceding claim, wherein said first material is substantially non-stretchable.

5. The pad of any preceding claim, wherein said second material is one or more selected from the group comprising: an elastomer; a gel; a foam; a low-density closed-cell polymer foam; substantially stretchable; pliant and elastic.

6. The pad of any preceding claim, wherein said second material and the skin of an orthopedic brace wearer have a higher friction coefficient relative to the first material and the skin of an orthopedic brace wearer.

7. The pad of any preceding claim, wherein said second material has a lower heat distortion temperature relative to said first material.

8. The pad of any preceding claim, wherein the pliant material is cloth and the second material is pliant elastic foam.

9. A method of fabricating an orthopedic brace pad comprising:
energizing a laminate having a first sheet of a perforated first material and a second sheet of a second material abutting said first sheet to a sufficient degree to substantially soften said second material to a substantially deformable state;
applying sufficient pressure to said softened second sheet to extrude said second material through perforations in said perforated first material of said first sheet; and
de-energizing and de-pressurizing said laminate resulting in an orthopedic brace pad having a plurality of nubs formed from said second material, wherein said nubs and said first sheet comprise in combination an uneven skin contact surface of said pad, said nubs being raised relative to said first sheet.

10. The method of claim 9, wherein said laminate is energized by heating said laminate.

11. The method of claim 9 or 10, wherein said laminate is energized by heating said laminate to a temperature above the heat distortion temperature of said second material.

12. The method of claim 9, 10 or 11, wherein said first material is a perforated cloth.

13. The method of any of claims 9 to 12, wherein said second material is one or more selected from the group comprising: an elastomer; a gel; a foam; and a low-density closed-cell polymer foam.

14. The method of any of claims 9 to 13, wherein said second material has a lower heat distortion temperature relative to said first material.

15. The method of any of claims 9 to 14, wherein said second material and the skin of an orthopedic brace wearer have a higher friction coefficient relative to the first material and the skin of an orthopedic brace wearer.
